# EUROPEAN PATENT APPLICATION

(11) **EP 4 410 203 A2**
(43) Date of publication of application: **07.08.2024**
(21) Application number: 23203803.4
(22) Date of filing: 16.10.2023
(51) Int. Cl.: A61B 5/16, A61B 5/00, G16H 10/20

(54) **PSYCHOLOGICAL TRAIT ESTIMATION SYSTEM AND METHOD**

(30) Priority: 02.02.2023 JP 2023014642
(71) Applicant: Hitachi, Ltd., Tokyo 100-8280 (JP)
(72) Inventor: NUMATA, Takashi, Tokyo, 100-8280 (JP); NAKAMURA, Toshiteru, Tokyo, 100-8280 (JP)
(74) Representative: MERH-IP Matias Erny Reichl Hoffmann Patentanwälte PartG mbB

(57) **Abstract**

A psychological trait estimation system provides a user with information that prompts an action for a purpose different from psychological trait estimation, receives, from an apparatus including one or a plurality of sensors, measurement data regarding an action prompted by the provided information and taken by the user, and specifies data representing designation of a user intention and data representing a relevant action based on the measurement data. The action prompted by information includes designation of a user intention and relevant actions that are all or some actions except the designation of the user intention. The system estimates a psychological trait of the user based on data representing a relevant action for one or each of a plurality of relevant actions, and outputs data representing the psychological trait having been estimated.

## Description

### BACKGROUND OF THE INVENTION

### 1. Field of the Invention

The present invention relates generally to a technique for estimation of a psychological trait.

### 2. Description of the Related Art

One example of psychological trait is personality. As a personality estimation technique, for example, techniques disclosed in US 2007/0271098 and US 10,957,306 are known. The technique disclosed in US 2007/0271098 estimates the personality of a user based on utterance data of the user. The technique disclosed in US 10,957,306 estimates the personality of a user based on text data in addition to utterance data of the user.

### SUMMARY OF THE INVENTION

It is conceivable to construct a technique (e.g., software or a system in which the software is implemented) for receiving, from one or a plurality of users, answer data representing an answer to a question, and for estimating an individual problem or an organizational problem based on the answer data. In order to solve an estimated problem, it is necessary for the user (individual user or each user belonging to an organization in which the problem is estimated) to take an action necessary for solving the problem. However, whether or not the user takes a necessary action is affected by the personality of the user.

Therefore, it is conceivable to estimate the personality of the user using the technique disclosed in US 2007/0271098 or US 10,957,306.

However, in the technique disclosed in any of US 2007/0271098 and US 10,957,306, the user needs to take another action such as utterance for personality estimation in addition to answering a question prepared for a purpose different from personality estimation. Not all users necessarily take such another action, and hence personality is not always estimated for all users.

Other than the individual problem or the organizational problem, there is a matter that is desired to be solved or improved by an action of one or each of a plurality of users. The user is provided with information that prompts an action, and the matter is estimated based on a user action (e.g., an answer to a question) in response to the provision. In order to solve or improve the estimated matter, it is necessary for each user to take a necessary action. However, not all users necessarily take actions different from the prompted action.

A psychological trait estimation system is constructed. A system provides a user with information that prompts an action for a purpose different from psychological trait estimation, receives, from an apparatus including one or a plurality of sensors, measurement data regarding an action prompted by the provided information and taken by the user, and specifies user intention data and relevant action data based on the measurement data. The action prompted by information includes designation of a user intention and relevant actions that are all or some actions except the designation of the user intention. The user intention data is data representing a designated user intention. The relevant action data is data representing a relevant action. A system estimates a psychological trait of the user based on relevant action data for one or each of a plurality of relevant actions, and outputs estimated psychological trait data that is data representing the psychological trait having been estimated.

According to the present invention, it is possible to estimate a psychological trait of a user without the user taking an action different from an action prompted by information provided for a purpose different from psychological trait estimation.

### BRIEF DESCRIPTION OF THE DRAWINGS

Fig. 1 illustrates a configuration example of an entire system according to an embodiment;
Fig. 2 illustrates data stored in a storage apparatus and functions of an arithmetic apparatus;
Fig. 3 illustrates a transition example of a question answer UI;
Fig. 4 illustrates an example of calculation of one evaluation index;
Fig. 5 illustrates an example of a keyword table; and
Fig. 6 illustrates an example of a practical application of a psychological trait estimation system.

### DESCRIPTION OF THE PREFERRED EMBODIMENTS

In the following description, the "interface apparatus" may be one or more interface devices. The one or more interface devices may be at least one of the following.
- An input/output interface apparatus that is one or more I/O interface devices. An input/output (I/O) interface device is an interface device for at least one of the I/O device and a remote display computer. The I/O interface device for the display computer may be a communication interface device. The at least one I/O device may be any of a user interface device, e.g., an input device such as a keyboard and a pointing device, and an output device such as a display device.
- A communication interface apparatus that is one or more communication interface devices. The one or more communication interface devices may be one or more communication interface devices of the same type (e.g., one or more network interface cards (NIC)) or two or more communication interface devices of different types (e.g., an NIC and a host bus adapter (HBA)).

In the following description, the "memory" is one or more memory devices that are an example of one or more storage devices, and may typically be a main storage device. The at least one memory device in the memory may be a volatile memory device or a nonvolatile memory device.

In the following description, the "persistent storage apparatus" may be one or more persistent storage devices that are an example of one or more storage devices. The persistent storage device may typically be a nonvolatile storage device (e.g., an auxiliary storage device), and may specifically be, for example, a hard disk drive (HDD), a solid state drive (SSD), a non-volatile memory express (NVME) drive, or a storage class memory (SCM) .

In the following description, the "storage apparatus" may be at least a memory of a memory and a persistent storage apparatus.

In the following description, the "processor" may be one or more processor devices. The at least one processor device may typically be a microprocessor device such as a central processing unit (CPU), but may also be a processor device of another type such as a graphics processing unit (GPU). The at least one processor device may be single-core or multi-core. The at least one processor device may be a processor core. The at least one processor device may be a processor device in a broad sense such as a circuit (e.g., a field-programmable gate array (FPGA), a complex programmable logic device (CPLD), or an application specific integrated circuit (ASIC)) that is an aggregate of gate arrays in a hardware description language for performing a part or the entirety of processing.

In the following description, a function will sometimes be described with an expression "yyy section", but the function may be implemented by executing one or more computer programs by a processor, may be implemented by one or more hardware circuits (e.g., FPGA or ASIC), or may be implemented by a combination thereof. In a case where the function is implemented by the processor executing a program, determined processing is performed using the storage apparatus and/or the interface apparatus as appropriate, and thus, the function may be at least a part of the processor. The processing described with the function as a subject may be processing performed by a processor or an apparatus including the processor. The program may be installed from a program source. The program source may be, for example, a program distribution computer or a computer-readable storage medium (e.g., a non-transitory storage medium). The description of each function is an example, and a plurality of functions may be put together into one function or one function may be divided into a plurality of functions.

In the following description, in a case where the same kind of elements are described without distinction, common reference signs among the reference signs may be used, and in a case where the same kind of elements are distinguished, reference signs may be used.

Hereinafter, an embodiment will be described. Note that in the following embodiment, personality can be adopted as an example of the psychological trait, but other types of psychological trait may be adopted. Personality has a plurality of personality components, for example, neuroticism, openness to experience, conscientiousness, extraversion, and agreeableness. Estimation of the psychological trait may include calculation of an evaluation value (score) for each personality component.

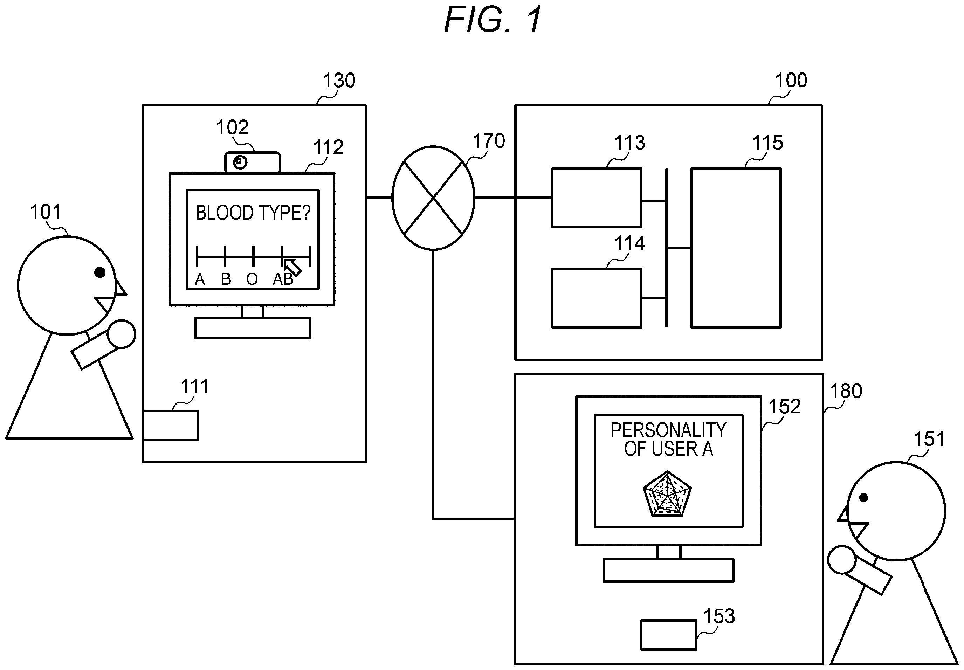

Fig. 1 illustrates a configuration example of an entire system according to the embodiment.

A psychological trait estimation system 100 communicates with a user apparatus 130 and an administrator apparatus 180 via a communication network 170. The communication network 170 is, for example, the Internet, a wide area network (WAN), or a local area network (LAN).

The user apparatus 130 is an information processing terminal of a user 101, for example, a computer such as a personal computer or a smartphone. The user apparatus 130 includes one or a plurality of sensors for measuring an action of the user 101 and a display device 112. The one or the plurality of sensors are, for example, a camera 102 and a mouse 111. The mouse 111 is an example of a pointing device. The display device 112 may be a touchscreen. The user 101 may be a user as a member of an organization.

The administrator apparatus 180 is an information processing terminal of an administrator 151, for example, a computer such as a personal computer or a smartphone. The administrator apparatus 180 includes an input device 153 and a display device 152. The administrator 151 may be a member of the organization to which the user 101 belongs and a person who formulates a measure for solving an organizational problem.

The psychological trait estimation system 100 includes an interface apparatus 113, a storage apparatus 114, and an arithmetic apparatus 115 connected to them.

The interface apparatus 113 communicates with the user apparatus 130 and the administrator apparatus 180 via the communication network 170. The storage apparatus 114 stores a computer program executed by the arithmetic apparatus 115 and data input and output by the arithmetic apparatus 115. The arithmetic apparatus 115 is a processor and executes a computer program.

The arithmetic apparatus 115 provides the user apparatus 130 with information that prompts the user 101 to take an action for a purpose different from the psychological trait estimation. That is, the "information" mentioned here is information prepared for a purpose different from the psychological trait estimation. In the present embodiment, the information includes one (or a plurality of) question and a plurality of options for answering the question, and provision of the information is display of the question and the plurality of options. The information may include a question but needs not include an option, and provision of the information may be display of the question (e.g., input of the answer may be voice input). The "question" provided in the present embodiment is a question prepared for a purpose different from the psychological trait estimation, such as a question prepared for estimating an organizational problem.

The arithmetic apparatus 115 receives, from one or a plurality of sensors through the interface apparatus 113, measurement data regarding an action taken by the user 101 prompted by the provided information, the measurement data based on measurement by the one or the plurality of sensors. Examples of the one or the plurality of sensors are the mouse 111 and the camera 102 in the present embodiment. The measurement data includes measurement data of the mouse 111 and measurement data of the camera 102. In the following description, measurement data of the mouse 111 will be mainly taken as an example.

Based on the measurement data, the arithmetic apparatus 115 specifies the user intention data and the relevant action data. The "action prompted by information" includes designation of a user intention and relevant actions that are all or some actions except the designation of the user intention. In the present embodiment, the designation of the user intention is to answer a question (in other words, for example, an answer reaction to a digital questionnaire), but the designation of the user intention may vary depending on information of the provision target. In the present embodiment, the relevant actions are all or some actions from when a question is provided to answering the question, but the relevant actions may also vary depending on designation of the user intention depending on information of the provision target. The user intention data is data representing a designated user intention. The relevant action data is data representing a relevant action.

The arithmetic apparatus 115 estimates the psychological trait of the user 101 based on relevant action data for one or each of a plurality of relevant actions. Specifically, for example, each time a question is displayed, the user 101 answers the displayed question, and there is a relevant action for each set of question and answer. For each of the plurality of relevant actions, there is relevant action data, and the arithmetic apparatus 115 estimates the psychological trait of the user 101 based on the relevant action data of the plurality of relevant actions.

The arithmetic apparatus 115 outputs estimated psychological trait data that is data representing the estimated psychological trait. For example, the arithmetic apparatus 115 transmits the estimated psychological trait data to the administrator apparatus 180, and the administrator apparatus 180 displays, on the display device 152, the psychological trait represented by the estimated psychological trait data. Due to this, the administrator 151 knows the estimated psychological trait of the user 101.

According to the present embodiment, it is possible to estimate the psychological trait of the user 101 without the user 101 taking an action different from an action (e.g., an answer to a question) prompted by information prepared and provided for a purpose different from the psychological trait estimation. Specifically, according to the present embodiment, it is not necessary to prepare information such as a question dedicated to psychological trait estimation, and the user 101 does not need to take the above-described "another action", for example, an action for information such as a question dedicated to the psychological trait estimation.

Note that the arithmetic apparatus 115 may output the user intention data to a computer system that performs processing for original use (in other words, a purpose different from the psychological trait estimation) based on the user intention.

Hereinafter, the present embodiment will be described in detail.

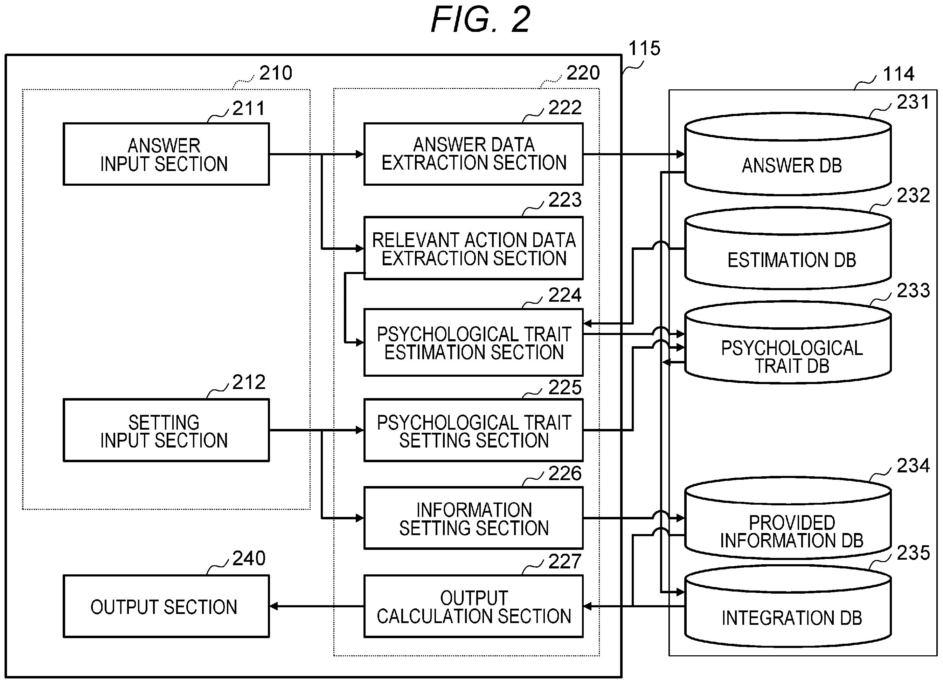

Fig. 2 illustrates data stored in the storage apparatus 114 and functions of the arithmetic apparatus 115. In the present embodiment, "DB" is an abbreviation for database. The data needs not be structured data such as a database.

The storage apparatus 114 stores an answer DB 231, an estimation DB 232, a psychological trait DB 233, a provided information DB 234, and an integration DB 235. The answer DB 231 is a DB that stores answer data (data representing an answer to a question). The estimation DB 232 is a DB that stores data (e.g., a model such as one or a plurality of regression equations) used for estimation of the psychological trait. The psychological trait DB 233 is a DB that stores estimated psychological trait data (data representing the estimated psychological trait). The provided information DB 234 is a DB that stores provided information (in the present embodiment, information including a question and a plurality of options). The integration DB 235 is a DB that stores integrated data of the answer data and the estimated psychological trait data.

By executing a computer program, the arithmetic apparatus 115 implement an input section 210, an arithmetic section 220, and an output section 240. The input section 210 includes an answer input section 211 and a setting input section 212. The arithmetic section 220 includes an answer data extraction section 222, a relevant action data extraction section 223, a psychological trait estimation section 224, a psychological trait setting section 225, an information setting section 226, and an output calculation section 227.

Hereinafter, an example of functions implemented by the arithmetic apparatus 115 and processing performed in the present embodiment will be described.

The setting input section 212 receives setting data (data of setting target) from the administrator apparatus 180. Examples of the setting data include setting data regarding psychological trait (e.g., data representing a plurality of personality components) and setting data regarding provided information (e.g., data including a question and a plurality of options for each question). The psychological trait setting section 225 stores, in the psychological trait DB 233, the setting data regarding the psychological trait. The information setting section 226 stores, in the provided information DB 234, setting data regarding the provided information.

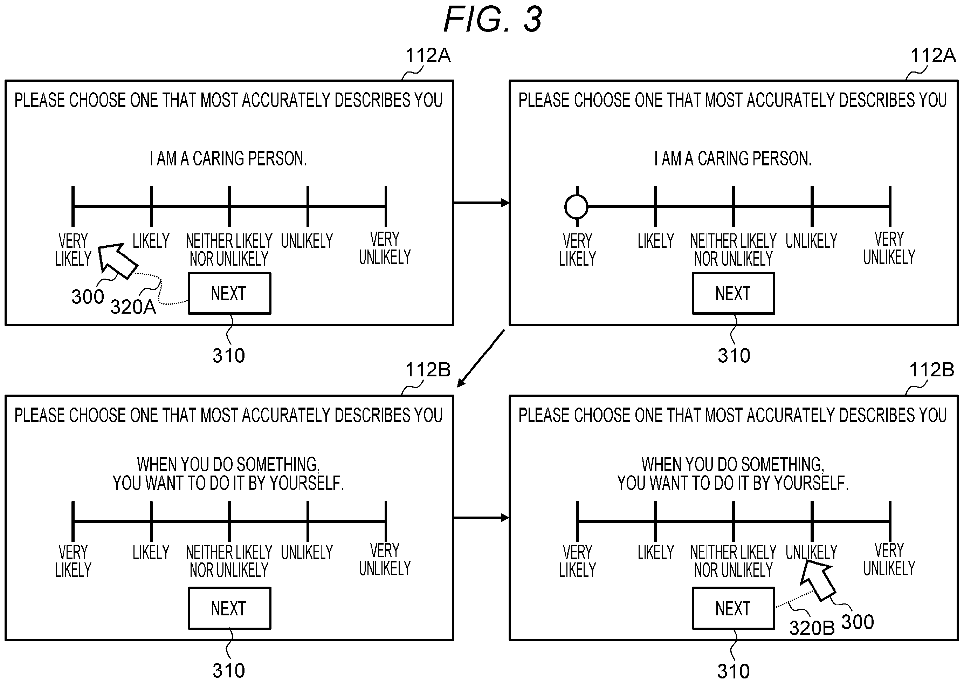

The output calculation section 227 acquires information of a provision target based on data in the provided information DB 234. For example, the output calculation section 227 generates a question answer user interface (UI) 112 illustrated in Fig. 3 based on the data in the provided information DB 234. The output section 240 provides the user apparatus 130 with information (the question answer UI 112) of the provision target. The information (the question answer UI 112) of the provision target is displayed on the display device 112. The information of the provision target may include, for example, an ID of a question, numbers of a plurality of options, and position information (e.g., coordinates) of each option.

The answer input section 211 receives, from the user apparatus 130, measurement data regarding an action taken by the user 101 prompted by the information provided from the output section 240. The measurement data includes answer data and relevant action data.

The answer data may include the ID of the question and the number of the option selected as an answer. The answer data extraction section 222 extracts the answer data from the measurement data and stores the extracted answer data in the answer DB 231. Note that the answer data may be specified from the measurement data (e.g., an answer may be specified from a relationship between cursor position information and option position information when a mouse cursor is pressed).

The relevant action may be an action up to reaching an answer (an example of designation of a user intention) to a question. Even if the answer is the same, the action until reaching the answer is affected by the psychological trait of the user 101. Since the relevant action data representing such an action is used for psychological trait estimation, it is expected to accurately estimate the psychological trait even if there is no dedicated question for psychological trait estimation or a user action. Specifically, for example, the relevant action data may include, for one or each of a plurality of action types, an action value representing a value of an action belonging to the action type. One or a plurality of action values included in the relevant action data may include at least one of a trajectory, coordinates, and a movement distance of the mouse cursor up to reaching the answer, the number of times of selection of options, and a time from the start of movement of the mouse cursor to the answer. Such an action value is considered to be affected by the psychological trait of the user 101, and hence, it is expected to accurately estimate the psychological trait. Note that the sensor may be another type of sensor, for example, a microphone, a motion sensor, or the like, instead of or in addition to at least one of the mouse 111 and the camera 102. In other words, the plurality of sensors may be two or more sensors of a pointing device, a touchscreen, a camera, a microphone, and a wearable sensor. The measurement data is not limited to a terminal operation such as an operation of the mouse 111 or a keyboard, and may include at least some data of data of a log related to a touchscreen operation, data such as a moving image and a voice during an answer to a question, and biological data such as an electroencephalogram, a cerebral blood flow, a heartbeat, or respiration. By using relevant action data specified based on measurement data of a plurality of sensors, it is expected to estimate the psychological trait more accurately.

The relevant action data extraction section 223 extracts the relevant action data from the measurement data. Note that the relevant action data may be specified from the measurement data (e.g., the relevant action data may be generated based on various values included in the measurement data). The relevant action data may be stored in the storage apparatus 114 for each answer to the question.

The psychological trait estimation section 224 estimates the psychological trait of the user 101 based on the relevant action data for one or each of a plurality of relevant actions, and stores the estimated psychological trait data representing the estimated psychological trait in the psychological trait DB 233.

The output calculation section 227 generates, for the user 101, integrated data of the estimated psychological trait data in the psychological trait DB 233 and the answer data (answer data for each answer to the question) in the answer DB 231, and stores the integrated data in the integration DB 235. The output section 240 outputs the integrated data of the user 101 to the administrator apparatus 180. The integrated data includes, for example, answer data for each answer to the question and estimated psychological trait data. The display device 152 displays the answer to each question and the estimated psychological trait.

The output data needs not be integrated data. For example, answer data (an example of user intention data) and estimated psychological trait data may be output at different timings. The output destination of the answer data and the output destination of the estimated psychological trait data may be the same or different. The estimated psychological trait data may be output to the user apparatus 130 instead of or in addition to the administrator apparatus 180. This enables the user 101 to know the estimated psychological trait of the user 101 by answering the question.

Fig. 3 illustrates a transition example of display in the user apparatus 130.

The question answer UI 112 is prepared for each question. The question answer UI 112 is typically a graphical user interface (GUI), and displays one question and a plurality of options. The question answer UI 112 includes a "NEXT" button 310 for designating proceeding to a next question. The position of the "NEXT" button 310 corresponds to the start position of a mouse cursor 300. That is, the following is performed for each of the first question to the final question. (x1) The question answer UI 112 is displayed, and the user 101 selects (clicks) a desired option from a plurality of options. The user 101 can select another option (correction of selection) instead of an option once selected before pressing (clicking) the "Next" button 310. (x2) The user 101 presses the "Next" button 310. If there is still a question, the processing returns to (x1).

According to the example illustrated in Fig. 3, the following is true. That is, the user 101 moves the mouse cursor 300 for selecting the leftmost option on a question answer UI 112A. A trajectory 320A of the mouse cursor 300 is measured in the user apparatus 130. The user 101 selects the leftmost option on the question answer UI 112A and presses the "Next" button 310. Then, the display transitions to display of the next question, specifically, a question answer UI 112B. The user 101 moves the mouse cursor 300 for selecting the rightmost option on the question answer UI 112B. A trajectory 320B of the mouse cursor 300 is measured in the user apparatus 130.

The plurality of prepared questions may be all questions unrelated to psychological trait or may include questions related to psychological trait. That is, no matter what question each of the plurality of questions is, the psychological trait of the user 101 can be estimated from the action including the answer to the question.

The psychological trait estimation section 224 calculates an evaluation value for one or each of a plurality of evaluation indices, and estimates the psychological trait of the user 101 based on the evaluation value of the one or the plurality of evaluation indices. For one or each of the plurality of evaluation indices, the psychological trait estimation section 224 calculates a plurality of parameter values based on the relevant action data, and calculates the evaluation value of the evaluation index based on the plurality of parameter values. From the plurality of calculated parameter values, components regarding perception and/or motion are relatively reduced based on an informational property that is a property of the information having been provided. Due to this, improvement of the estimation accuracy of the psychological trait is expected. Note that as an evaluation index, for example, at least one of an initial reaction time (time from when the question answer UI 112 is displayed to when the movement of the mouse cursor 300 starts), an answer time (total time from the display of the question answer UI 112 to answer completion), a cursor movement distance (movement distance of the mouse cursor 300 until an option is selected from the "NEXT" button 310), and the number of times of selection of answers (total number of times an option has been selected from when the question answer UI 112 is displayed until the "Next" button 310 is pressed) may be adopted.

The psychological trait estimation section 224 may calculate an evaluation value for one or each of the plurality of evaluation indices, and calculate an evaluation value (score) for each of a plurality of personality components based on the evaluation value of the one or the plurality of evaluation indices. Specifically, for example, one or more personality components are associated with each evaluation index, and the evaluation value calculated for an evaluation index may be reflected in each of the one or more personality components associated with the evaluation index. The estimated psychological trait may include an evaluation value calculated for each of the plurality of personality components. That is, the estimated psychological trait may include one or both of qualitative psychological trait and quantitative psychological trait.

For each of the plurality of evaluation indices, calculation of the evaluation index may be performed by a method corresponding to the evaluation index. For example, a model (e.g., a machine learning model) such as a regression equation may be prepared for each evaluation index, and the evaluation index may be calculated using the model.

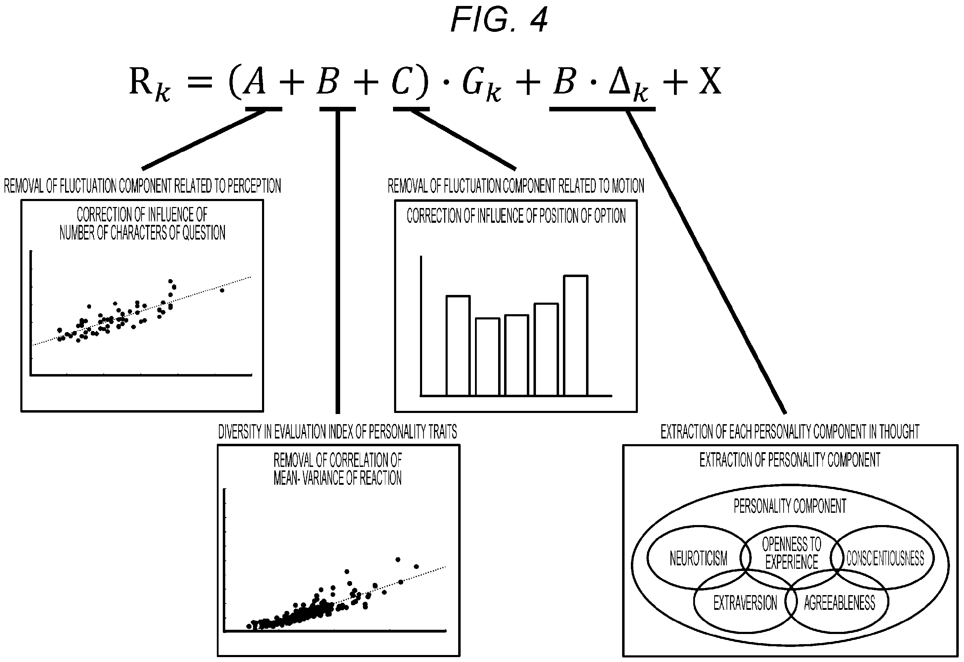

Fig. 4 illustrates an example of calculation of one evaluation index.

In Fig. 4, the evaluation index is an initial reaction time. An answer reaction model-based model of Miller et al. is adopted to calculate the evaluation value of the initial reaction time. In the model illustrated in Fig. 4, Rₖ is an evaluation value of the initial reaction time. A is a parameter (component) related to perception. B is a parameter related to thought. C is a parameter related to motion. X is a parameter related to error. Such a model may be prepared for each evaluation index.

The informational property includes a length of a question (an example of information). The "length of question" may be a time length of voice output in a case where a question is output by voice, but is the number of characters constituting the question in the present embodiment. In general, it is considered that the larger the number of characters, the longer it takes to understand the question, and hence the longer the initial reaction time tends to be regardless of psychological trait. The psychological trait estimation section 224 determines the value of A (value of the parameter related to perception) based on the length of the question. Specifically, for example, there is an initial reaction time for each of the plurality of questions, but the psychological trait estimation section 224 corrects each of the plurality of initial reaction times based on the number of characters of the question corresponding to the initial reaction time (in other words, the influence of the number of characters is reduced (e.g., removed) from the initial reaction time), and determines the value of A based on the plurality of corrected initial reaction times. Due to this, the value of A becomes appropriate, and hence, the accuracy of Rₖ, which becomes an element of psychological trait estimation, is improved, whereby the accuracy of the psychological trait estimation is expected to be improved.

The value of B (parameter value related to thought) is a parameter value based on a corrected standard deviation of an action value corresponding to the parameter value. Specifically, for example, the psychological trait estimation section 224 sets, as the value of B, a value based on a result obtained by removing the correlation between the mean value of the initial reaction time and the standard deviation from the correlation among the mean value of the initial reaction time, the standard deviation, and the psychological trait. Due to this, the value of B becomes appropriate, and hence, the accuracy of Rₖ, which becomes an element of psychological trait estimation, is improved, whereby the accuracy of the psychological trait estimation is expected to be improved.

The informational property includes a position of each of the plurality of options. In general, it is considered that the farther the position of the selected option is from the "Next" button 310, the longer the initial reaction time tends to be. The psychological trait estimation section 224 determines the value of C (value of the parameter related to motion) based on the position of the selected option. Specifically, for example, there is an initial reaction time for each of the plurality of questions, but the psychological trait estimation section 224 corrects each of the plurality of initial reaction times based on the position of the option corresponding to the initial reaction time (in other words, the influence of the option position is reduced (e.g., removed) from the initial reaction time), and determines the value of A based on the plurality of corrected initial reaction times. Due to this, the value of C becomes appropriate, and hence, the accuracy of Rₖ, which becomes an element of psychological trait estimation, is improved, whereby the accuracy of the psychological trait estimation is expected to be improved.

The value of B•Δₖ in the model illustrated in Fig. 4 may be a value determined based on the personality component. For example, the value of Δₖ may be determined based on a ratio of questions belonging to the personality component to a plurality of questions.

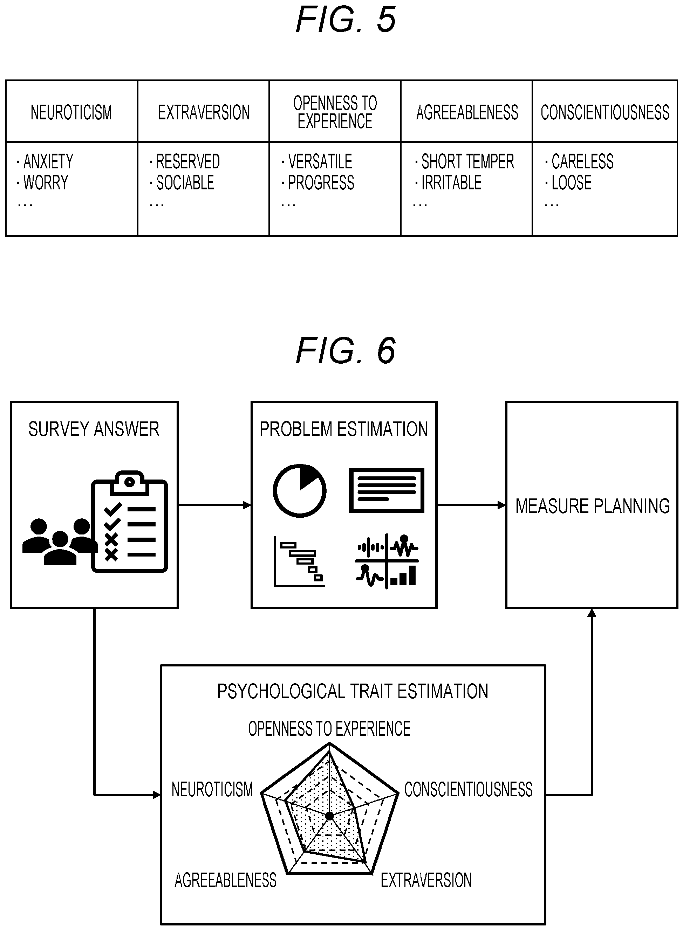

The "questions belonging to the personality component" are, for example, questions including keywords belonging to the personality component. Specifically, for example, as illustrated in Fig. 5, a keyword table in which keywords are registered for each personality component may be stored in the estimation DB 232 in advance, for example. The keyword table is an example of data representing keywords related to psychological trait, and represents keywords for each personality component, for example. In estimation of the psychological trait of the user 101, the psychological trait estimation section 224 may set the weight of a question including the keyword related to the psychological trait to be relatively higher than the weight of a question not including the keyword related to the psychological trait. For example, the psychological trait estimation section 224 may use relevant action data regarding an answer to a question including a keyword related to the psychological trait for estimation of the psychological trait, and needs not use relevant action data regarding an answer to a question not including a keyword related to the psychological trait. Due to this, it is expected to accurately estimate the psychological trait.

The processing using the keyword table may be the following processing instead of or in addition to evaluation value calculation using the model illustrated in Fig. 4. That is, the psychological trait estimation section 224 may determine, for each of the plurality of questions represented by the provided information DB 234, whether or not the question includes a keyword represented by the keyword table, and classify the question based on the determination result. For example, if the question includes one or a plurality of keywords, in the classification of the question, the personality component to which the keyword belongs may be associated with the question. A plurality of personality components may be associated with one question, or a personality component to which the most keywords among the keywords included in the question belong may be associated with one question. A question associated with a personality component may be a question with a relatively high weight. When calculating the evaluation value for each personality component, the psychological trait estimation section 224 may use relevant action data regarding an answer to a question associated with the personality component, and needs not use relevant action data regarding an answer to a question not associated with the personality component. Due to this, it is expected to calculate an appropriate evaluation value for each personality component.

Fig. 6 illustrates an example of a practical application of the psychological trait estimation system 100.

A survey answer is performed by each of a plurality of users belonging to the same organization. Specifically, for example, a plurality of users each answer each of a plurality of questions prepared for estimation of an organizational problem. Data representing a survey answer of each user (data representing an answer for each question) is output by the output section 240, and the data is input to an organizational problem estimation function (function of estimating an organizational problem). The organizational problem estimation function estimates an organizational problem based on data representing the survey answer of each user, and outputs estimated organizational problem data, which is data representing the estimated organizational problem, to a measure planning function (function of taking planning a measure). At least one of the organizational problem estimation function and the measure planning function may be a function in the psychological trait estimation system 100 or a function outside the psychological trait estimation system 100 (e.g., a physical computer system or a logical computer system such as a cloud computing service).

For each user, the relevant action data extraction section 223 extracts, from the measurement data, relevant action data for each answer to the question regarding the survey answer (answer to each question), and the psychological trait estimation section 224 estimates the psychological trait based on the relevant action data for each answer. The output section 240 outputs the estimated psychological trait data of each user to the measure planning function.

The measure planning function plans a measure for solving the estimated organizational problem based on the estimated organizational problem data. The measure includes an action that should be taken by the user belonging to the organization. Based on the psychological trait represented by the estimated psychological trait data corresponding to the user, the measure planning function generates, for each user belonging to the organization, a proposal for the user to take the action that should be taken by the user. The proposal is an example of content based on the psychological trait. That is, the measure planning function is an example of a function of determining content to be output based on the psychological trait.

According to such a practical application, the possibility that each user takes an action for solving the organizational problem can be increased by a technical means that is the psychological trait estimation system 100.

The psychological trait estimation system 100 can also be applied to other practical applications. For example, the psychological trait estimation system 100 may determine content to be output based on an estimated psychological trait, or may output the estimated psychological trait data to a function to determine content to be output based on the psychological trait. This enables the user to receive content matching the psychological trait of the user.

While one embodiment has been described above, this is an example for description of the present invention, and it is not intended to limit the scope of the present invention only to this embodiment. The present invention can be carried out in various other forms.

For example, the psychological trait estimation section 224 may perform principal component analysis with a component correlated with a psychological trait category specified in advance as a principal component based on a plurality of action values included in relevant action data of the answer for each question, and estimate the psychological trait of the user 101 based on the result of the principal component analysis. Due to this, highly accurate psychological trait estimation based on a higher correlation is expected. Specifically, for example, the following may be performed.

That is, the psychological trait estimation section 224 may extract a plurality of action features based on a plurality of action values of relevant action data of the answers to a plurality of questions. The plurality of action features may be at least some of a mean and a standard deviation of answer times to each question and answer confirmation times, an answer time to the first question (time after correction of the length of a question sentence), a mean of initial answer times, and a standard deviation (after correction of the distance between the length of the question sentence and the answer option), a mean and a standard deviation of cursor movement amounts, a movement amount to the first question, or the like. The plurality of action features may include at least some of the evaluation values of the plurality of evaluation indices described above.

The psychological trait estimation section 224 may perform calculation of a direction in which the variance from the mean value becomes maximum (first principal component), calculation of a portion orthogonal to the first principal component and having the maximum variance (second principal component), and repeat of the calculation of the portion orthogonal to the immediately preceding principal component and having the maximum variance for the data dimension (nth principal component). The direction in which a vector wₖ having the largest inner product with respect to data xᵢ has the largest variance may be wₖ₌₁ = argmax {Σᵢ (x·w)²}, tₖ₍ᵢ₎ = xᵢ·wₖ, and a vector t₍ᵢ₎ = (t₁,..., tₖ)₍ᵢ₎ may be a principal component score.

## Claims

1. A psychological trait estimation system comprising:
an interface apparatus connected to a user apparatus that is an apparatus including one or a plurality of sensors; and
an arithmetic apparatus connected to the interface apparatus, wherein
the arithmetic apparatus provides the user apparatus with information that prompts a user to take an action for a purpose different from psychological trait estimation,
the arithmetic apparatus receives, from the user apparatus through the interface apparatus, measurement data based on measurement by the one or the plurality of sensors related to an action taken by the user prompted by the information having been provided,
the arithmetic apparatus specifies user intention data and relevant action data based on the measurement data,
the action prompted by the information includes designation of a user intention and a relevant action that is all or some actions except the designation of the user intention,
the user intention data is data representing the user intention having been designated,
the relevant action data is data representing a relevant action,
the arithmetic apparatus estimates a psychological trait of the user based on relevant action data for one or each of a plurality of relevant actions, and
the arithmetic apparatus outputs estimated psychological trait data that is data representing the psychological trait having been estimated.

2. The psychological trait estimation system according to claim 1, wherein
the relevant action is an action up to reaching designation of the user intention.

3. The psychological trait estimation system according to claim 2, wherein
provision of the information is display of the information,
the relevant action data includes an action value representing a value of an action belonging to the action type for one or each of a plurality of action types, and
one or a plurality of action values included in the relevant action data include at least one of a trajectory, coordinates, a movement distance of a cursor until reaching designation of the user intention, and a time from movement start to designation of the user intention.

4. The psychological trait estimation system according to claim 1, wherein
the plurality of sensors are two or more sensors of a pointing device, a touchscreen, a camera, a microphone, and a wearable sensor.

5. The psychological trait estimation system according to claim 1, wherein
the relevant action data includes an action value representing a value of an action belonging to the action type for one or each of a plurality of action types,
the arithmetic apparatus calculates an evaluation value for one or each of a plurality of evaluation indices, and estimates a psychological trait of the user based on the evaluation value of the one or the plurality of evaluation indices,
for each of the one or the plurality of evaluation indices,
the arithmetic apparatus calculates a plurality of parameter values based on the relevant action data, and calculates an evaluation value of the evaluation index based on the plurality of parameter values, and
from the plurality of calculated parameter values, components regarding perception and/or motion are relatively reduced based on an informational property that is a property of the information having been provided.

6. The psychological trait estimation system according to claim 5, wherein
the relevant action data includes an action value representing a value of an action belonging to the action type for one or each of a plurality of action types, and
among the plurality of parameter values having been calculated, a parameter value related to thought is a parameter value based on a corrected standard deviation of an action value corresponding to the parameter value.

7. The psychological trait estimation system according to claim 5, wherein
the informational property includes a length of the information.

8. The psychological trait estimation system according to claim 5, wherein
the information includes one or more questions and a plurality of options for an answer to each of the one or more questions,
provision of the information includes display of the plurality of options,
designation of the user intention is selection of at least one option of the plurality of options,
the relevant action data includes an action value representing a value of an action belonging to the action type for one or each of a plurality of action types,
one or a plurality of action values included in the relevant action data include at least one of a trajectory, coordinates, and movement distance of a cursor until reaching designation of the user intention, a number of times of selection of options, and a time from movement start to designation of the user intention, and
the informational property includes a position of each of the plurality of options.

9. The psychological trait estimation system according to claim 1, wherein
the relevant action data includes, for each of a plurality of action types, an action value representing a value of an action belonging to the action type, and
based on a plurality of action values included in the relevant action data, the arithmetic apparatus performs principal component analysis with a component correlated with a psychological trait category specified in advance as a principal component, and estimates a psychological trait of the user based on a result of the principal component analysis.

10. The psychological trait estimation system according to claim 1, wherein
the information includes one or more questions, and
in estimation of a psychological trait of the user, the arithmetic apparatus sets a weight of a question including a keyword related to a psychological trait to be relatively higher than a weight of a question not including a keyword related to a psychological trait.

11. The psychological trait estimation system according to claim 1, wherein
the arithmetic apparatus outputs the estimated psychological trait data to a function of determining content to be output based on the psychological trait.

12. A psychological trait estimation method, the method comprising:
providing, by a computer, information that prompts a user to take an action for a purpose different from psychological trait estimation to a user apparatus that is an apparatus including one or a plurality of sensors,
receiving, by a computer, from the user apparatus, measurement data based on measurement by the one or the plurality of sensors related to an action taken by the user prompted to the information having been provided, and
specifying, by a computer, user intention data and relevant action data based on the measurement data,
wherein the action prompted by the information includes designation of a user intention and a relevant action that is all or some actions except the designation of the user intention,
wherein the user intention data is data representing the user intention having been designated, and
wherein the relevant action data is data representing a relevant action,
estimating, by a computer, a psychological trait of the user based on relevant action data for one or each of a plurality of relevant actions, and
outputting, by a computer, estimated psychological trait data that is data representing the psychological trait having been estimated.

13. A computer program that causes a computer to execute
providing information that prompts a user to take an action for a purpose different from psychological trait estimation to a user apparatus that is an apparatus including one or a plurality of sensors,
receiving, from the user apparatus, measurement data based on measurement by the one or the plurality of sensors related to an action taken by the user prompted to the information having been provided,
specifying user intention data and relevant action data based on the measurement data,
wherein the action prompted by the information includes designation of a user intention and a relevant action that is all or some actions except the designation of the user intention,
wherein the user intention data is data representing the user intention having been designated, and
wherein the relevant action data is data representing a relevant action, and
estimating a psychological trait of the user based on relevant action data for one or each of a plurality of relevant actions, and
outputting estimated psychological trait data that is data representing the psychological trait having been estimated.
